# EUROPEAN PATENT APPLICATION

(11) **EP 3 628 263 A1**
(43) Date of publication of application: **01.04.2020**
(21) Application number: 18197293.6
(22) Date of filing: 27.09.2018
(51) Int. Cl.: A61B 34/20

(54) **GUIDANCE IN LUNG INTERVENTION PROCEDURES**

(71) Applicant: Koninklijke Philips N.V., 5656 AG Eindhoven (NL)
(72) Inventor: MUELLER, Manfred, 5656 AE Eindhoven (NL); SABCZYNSKI, Jörg, 5656 AE Eindhoven (NL); REICH, Christian, 5656 AE Eindhoven (NL)
(74) Representative: de Haan, Poul Erik

(57) **Abstract**

The present invention relates to guidance in lung intervention procedures like lung biopsy. In order to provide further improved needle guidance, a device (10) for guidance in a lung intervention procedure comprises an input unit (12), a data storage unit (14), a data processing unit (16), and an output unit (18). The input unit provides current tracking data of at least one first reference marker relating to a subject's abdomen area and of at least one second reference marker relating to the subject's thorax area. The data storage unit provides a 3D motion model of a torso of the subject comprising at least the lung and a part of the abdomen area. The 3D motion model is based on pre-operationally acquired image data of the subject. The data processing unit registers the reference markers with the 3D motion model, and determines a current motion of the reference markers. The data processing unit also transfers the current motion of the reference markers to the 3D motion model, and adapts the 3D motion model based on the transferred current motion. The data processing unit adjusts positions of predetermined lung structures within the 3D motion model, and adjusts a planned trajectory of an interventional device according to the adjusted positions to provide a live-adjusted trajectory path. The output unit is configured to provide the adjusted trajectory for the lung intervention procedure. In an example, a guidance system (50) is provided that comprises such device and also a marker tracking device (54) that tracks at least one first reference marker relating to the subject's abdomen area and at least one second reference marker relating to the subject's thorax area and that provides the current tracking data.

## Description

### FIELD OF THE INVENTION

The present invention relates to lung intervention procedures like lung biopsy, and relates in particular to a device for guidance in a lung intervention procedure, to a guidance system for lung intervention procedures, and to a method for guidance in lung intervention procedures.

### BACKGROUND OF THE INVENTION

Among lung diseases, lung cancer is a major cause of death and a respective effort is undertaken for diagnostic and treatment of lung cancer. For example, systematic lung cancer screening programs are implemented. Diagnosis and management of solitary pulmonary nodules (SPNs) have become an increasing clinical problem, with prevalence of SPNs as high as 50% in lung cancer screening studies of high risk smokers. Lesions may be biopsied and/or surgically excised. An example for one of many steps, biopsies may be applied, for example in a percutaneous or endobronchial way. In both cases, a clinician may navigate a suitable biopsy device into the lesion to retrieve a tissue sample. However, these biopsies may be subject to failure, because the physician is not really able to correctly target the lesion. One important factor for such interventions like a lung biopsy is the motion of the region of interest caused by breathing. Instructing a subject to hold breathing, or at least reduce breathing, for a certain time is an option, but not always really applicable. Hence, success rates for biopsies may differ widely depending on the approach used, the guidance methodology, and also lesion size, type and location. To improve targeting of the lesion, placement of markers inside of a lesion in preparation for surgical removal or radiation treatment may be provided. However, also in this case the clinician will need to place the tip of a needle (or similar device) inside the lesion. For example, for providing guidance during biopsy procedures, WO 2015 079456 A2 describes to calculate a needle trajectory, to overlay the calculated needle trajectory and needle angles information on a display monitor, to align a marker marking a needle entry point by moving a camera or the couch, based on the calculated needle trajectory and needle angles displayed on the display screen, to adjust the needle orientation using real time image displayed on the display screen, after adjusting the needle insertion point, and to compute a needle insertion depth with the processor to guide a medical practitioner to reach a target location, after matching the needle orientation. As another example, for robotic biopsy, US 2015 0073259 A1 describes image-guidance for performing needle biopsy on a moving lung nodule of a body. CT images of the lung nodule are obtained to generate a motion model, based on which an optimal needle advancing path is determined. The motion of the lung nodule and the motion of a fiducial marker attached to the body are correlated. The motion of the fiducial marker is tracked and monitored by a camera to determine a position of the lung nodule based on the correlation. A time for advancing the needle is determined based on a motion attribute of the reference. The needle is advanced by a robotic needle manipulator at the predetermined time along the path to accomplish the needle placement. However, timing may be burdensome for the operator.

### SUMMARY OF THE INVENTION

There may thus be a need to provide further facilitated and improved needle guidance.

The object of the present invention is solved by the subject-matter of the independent claims; further embodiments are incorporated in the dependent claims. It should be noted that the following described aspects of the invention apply also for the device for guidance in a lung intervention procedure, for the guidance system for lung intervention procedures and for the method for guidance in lung intervention procedures.

According to the present invention, a device for guidance in a lung intervention procedure is provided. The device comprises an input unit, a data storage unit, a data processing unit and an output unit. The input unit is configured to provide current tracking data of at least one first reference marker relating to a subject's abdomen area and of at least one second reference marker relating to the subject's thorax area. The data storage unit is configured to provide a 3D motion model of a torso of the subject comprising at least the lung and a part of the abdomen area. Further, the 3D motion model is based on pre-operationally acquired image data of the subject. The data processing unit is configured to register the reference markers with the 3D motion model and to determine a current motion of the reference markers. The data processing unit is also configured to transfer the current motion of the reference markers to the 3D motion model and to adapt the 3D motion model based on the transferred current motion. The data processing unit is further configured to adjust positions of predetermined lung structures within the 3D motion model and to adjust a planned trajectory of an interventional device according to the adjusted positions to provide a live-adjusted trajectory path. The output unit is configured to provide the adjusted trajectory for the lung intervention procedure.

This provides improved needle guidance, since the complete planned trajectory is updated according to the current situation regarding the motion of the subject.

Since the input unit is configured to provide current tracking data, i.e. live data, the "device for guidance in a lung intervention procedure" can thus be referred to as "device for real-time guidance in a lung intervention procedure".

It is noted that the term "reference marker" refers to a pre-determined feature suitable for acting as (spatial) reference.

In an example, the term "reference markers" thus refers to separate reference markers provided to be attached to a subject, or located in a temporarily fixed manner at/on/inside the subject.

In another example, the term "reference markers" thus also refers to reference landmarks of the subject itself, i.e. natural landmarks, e.g. an anatomical landmark, or also artificial landmarks like a prosthesis of the subject visible in the respective medical imaging. An example of natural landmarks are the sternum and the navel. An option in X-ray is to use the sternum and the diaphragm.

The term "current" in "current tracking data" relates to an actual or present situation, e.g. a live situation. "Current" thus relates to a simultaneous, or near-simultaneous data processing. In other words, the "current tracking data" represents live or real-time tracking data. It is noted that the terms "live" and "real-time" also comprise minor delays in the data processing procedure.

In an example, the registration of the reference markers is based on provided location identification, which allow the spatial alignment of the reference markers positions and the respective locations of the 3D motion model.

According to an example, the 3D motion model comprises a first 3D motion sub-model of the diaphragm and a second motion sub-model of the thorax. The data processing unit is configured to transfer the current motion of the at least one first reference marker relating to the subject's abdomen area to the first 3D motion sub-model of the diaphragm and to transfer the current motion of the at least one second reference marker relating to the subject's thorax area to the second 3D motion sub-model of the thorax. The data processing unit is also configured to adapt the first and second 3D motion sub-models based on the transferred current motion. Further, the data processing unit is configured to adjust the positions of predetermined lung structures within the 3D motion sub-models, and to adjust the planned trajectory of an interventional device according to the adjusted positions to provide the live-adjusted trajectory path.

According to an example, the interventional device is a biopsy needle and the planned trajectory is a planned needle trajectory. The data processing unit is configured to adjust the planned needle trajectory according to the current motion of the subject. The planned needle trajectory is adjusted for at least one of the group of insertion point, trajectory angle and spatial depth.

In an example, the motion of the at least one first reference marker is assigned to the first motion sub-model of the diaphragm and the motion of the at least one second reference marker is assigned to the second motion sub-model of the thorax.

According to the present invention, also a guidance system for lung intervention procedures is provided. The system comprises a device for guidance in lung intervention procedures according to one of the preceding examples. The system also comprises a marker tracking device for tracking at least one first reference marker relating to the subject's abdomen area and at least one second reference marker relating to the subject's thorax area. The marker tracking device is configured to track the reference markers and to provide the current tracking data.

The term "marker tracking device" relates to a device for tracking reference markers that can be provided as (see above) separate reference markers or as reference landmarks, e.g. anatomical landmarks.

In an example, the marker tracking device is a "marker tracking device for tracking external reference markers", or an "external reference marker tracking device", for tracking at least one first external reference marker relating to the subject's abdomen area and at least one second external reference marker relating to the subject's thorax area. The marker tracking device is configured to track the external reference markers and to provide the current tracking data.

In another example, the marker tracking device is a "marker tracking device for tracking reference landmarks", or a "reference landmark tracking device", for tracking at least one first reference landmark relating to the subject's abdomen area and at least one second reference landmark relating to the subject's thorax area. The marker tracking device is configured to track the reference landmarks and to provide the current tracking data.

According to an example, it is further provided at least one first reference marker relating to the subject's abdomen area, and at least one second reference marker relating to the subject's thorax area.

In a first option, the reference markers are external markers configured to be provided temporarily on the subject. The at least one first reference marker is configured to be provided on the subject relating to the subject's abdomen area, and the at least one second reference marker is configured to be provided on the subject relating to the subject's thorax area.

In a second option, provided in addition or as an alternative, the reference markers are landmarks of a subject. The at least one first reference marker is a determined first landmark of the subject relating to the subject's abdomen area, and the at least one second reference marker is a determined second landmark of the subject relating to the subject's thorax area

In an example, pre-determined anatomical landmarks may be used as reference markers.

According to an example, it is further provided a camera system with at least one camera. The reference markers are tracked by the at least one camera.

According to an example, a projecting device is provided configured to project the live-adjusted trajectory path to the user.

According to an example, a handling device for operating the interventional device is provided configured to move the interventional device according to path data, and the live-adjusted trajectory path is provided to the handling device as updated path data.

According to the present invention, also a method for guidance in lung intervention procedures is provide. The method comprises the following steps:
a) a 3D motion model of a torso of a subject is provided comprising at least the lung and a part of the abdomen area; wherein the 3D motion model is based on pre-operationally acquired image data of the subject;
b) at least one first reference marker on the subject relating to the subject's abdomen area is identified; and at least one second reference marker on the subject relating to the subject's thorax area is identified;
c) the reference markers are registered with the 3D motion model;
d) the at least one first reference marker and the at least one second reference marker are tracked to determine a current motion of the reference markers;
e) the current motion of the reference markers is transferred to the 3D motion model and the 3D motion model is adapted based on the transferred current motion; and positions of predetermined lung structures within the 3D motion model are adjusted;
f) a planned trajectory of an interventional device is adjusted according to the adjusted positions to provide a live-adjusted trajectory path; and
g) the adjusted trajectory is provided for the lung intervention procedure.

According to an aspect, it is provided to continuously trace the position of anatomical or artificial markers placed on or inside the chest of the subject using tracking with camera-, X-ray-, ultrasound- or MR-based images. The lung deformation due to subject breathing is simulated, based on the location of these markers and a pre-procedure 3D image of the lungs and the abdomen. This could be a CT, MR, cone-beam CT (XperCT) or 3D ultrasound image. In an example, the markers are visible in this image as well to enable easy registration. Further, the position of important lung structures like lesions or major blood vessels is simulated based on tracking the movement of the markers in real-time. In an option, the simulation based on the real-time marker tracking is complemented (if needed) with supplementary real-time information such as physiological subject data, like pulse etc., or other moving anatomical landmarks. The planned or real trajectory of a device like a biopsy needle, biopsy device, etc. is then adjusted in real time based on the new location information so that the device can reach the target while avoiding sensitive structures.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a device for guidance in a lung intervention procedure.
Fig. 2 shows an example of a guidance system for lung intervention procedures.
Fig. 3a and 3b show schematic illustrations relating to motion due to breathing, wherein Fig. 3a refers to an initial state when inhaling and Fig. 3b refers to a moved state when exhaling with indicated adjusted trajectory.
Fig. 4 shows an example of a method for guidance in lung intervention procedures.

### DETAILED DESCRIPTION OF EMBODIMENTS

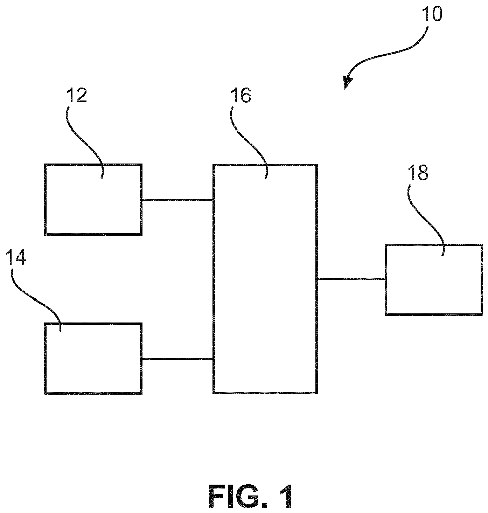

Fig. 1 shows an example of a device 10 for guidance in a lung intervention procedure. The device 10 comprise an input unit 12, a data storage unit 14, a data processing unit 16 and an output unit 18.

The input unit 12 is configured to provide current tracking data of at least one first reference marker relating to a subject's abdomen area and of at least one second reference marker relating to the subject's thorax area.

The data storage unit 14 is configured to provide a 3D motion model of a torso of the subject comprising at least the lung and a part of the abdomen area. The 3D motion model is based on pre-operationally acquired image data of the subject.

The data processing unit 16 is configured to register the reference markers with the 3D motion model, and to determine a current motion of the reference markers. The data processing unit 16 is also configured to transfer the current motion of the reference markers to the 3D motion model, and to adapt the 3D motion model based on the transferred current motion. The data processing unit 16 is further configured to adjust positions of predetermined lung structures within the 3D motion model, and to adjust a planned trajectory of an interventional device according to the adjusted positions to provide a live-adjusted trajectory path.

The output unit 18 is configured to provide the adjusted trajectory for the lung intervention procedure. The adjusted trajectory can be real-time or predictive for expected breathing motion.

In an option, not further shown, the 3D motion model comprises a first 3D motion sub-model of the diaphragm and a second motion sub-model of the thorax. The data processing unit 16 is configured to transfer the current motion of the at least one first reference marker relating to the subject's abdomen area to the first 3D motion sub-model of the diaphragm and to transfer the current motion of the at least one second reference marker relating to the subject's thorax area to the second 3D motion sub-model of the thorax. The data processing unit 16 is also configured to adapt the first and second 3D motion sub-models based on the transferred current motion. Further, the data processing unit 16 is configured to adjust the positions of predetermined lung structures within the 3D motion sub-models, and to adjust the planned trajectory of an interventional device according to the adjusted positions to provide the live-adjusted trajectory path.

The term "lung intervention procedure" relates to e.g. a lung biopsy procedure or other examination or operation procedure.

The term "interventional device" relates to a biopsy needle or other examination or operation device.

The term "subject" may also be referred to as individual. The "subject" may further also be referred to as patient, although it is noted that this term does not indicate whether any illness or disease is actually present with the subject.

In an example, not further shown, it is further provided a model-generating unit that is configured to generate motion models based on 3D image data by segmenting moving structures and assigning mechanical properties and boundary conditions to the moving structures.

For example, the planned trajectory is a biopsy needle insertion trajectory, also referred to as biopsy path.

A planned trajectory may be provided, for example, based on findings such as identifying a lesion of interest, and based on indications or instructions from a surgeon. A path can then be planned based on static image data of the subject. The tracking of the markers and transferring to the 3D model is used to achieve moving image data of the subject. This moving image data, i.e. current or live data, is used to adjust the planned path.

The thorax can also be referred to as chest or ribcage. The diaphragm can also be referred to as midriff.

The pre-operationally acquired image data can be provided, for example, as CT image data or as MRI image data or from another suitable imaging modality. In an example, a 3D image of the subject's chest is taken. Key structures like the diaphragm, a lesion, major blood vessels, major bronchi, lobes etc. should be visible and/or segmented.

The at least one first reference marker can be referred to as diaphragm marker, and the at least one second reference marker as thorax marker. The at least one first reference marker can also be referred to as first marker and the at least one second reference marker as second marker. In an example, two or more first reference markers are provided. In another example, two or more second reference markers are provided.

In an example, the reference markers are provided by temporary attachment to the subject's anatomy, i.e. the subject's body, before the pre-operationally image data of the subject is acquired. These pre-attached markers are optimized to be visible with an optical camera system as well as with the chosen 3D imaging modality. For example, the optical camera is provided as a camera attached to equipment above a subject support like a patient table, such as lighting equipment or a detector of an X-ray imaging modality. In an example, a time of flight camera is used for tracking the markers' motion.

The camera may be provided as part of an interventional system from Philips equipped with augmented-reality surgical navigation technology. Herein, high-resolution cameras are added to an interventional X-ray system for example to enhance spine, neuro or trauma surgery. Such system is also referred to as a "Light2C" system hereinafter. In an example, Light2C may help to increase the yield of (percutaneous) needle insertions by helping the clinician in adjusting the needle trajectory.

In another example, the registration of the reference markers with the 3D motion model is achieved by common landmarks or other anatomy structures visible, respectively present, in the tracking mode and the 3D motion model.

The predetermined lung structures may comprise lesions, major blood vessels or the like.

In an option, not further shown in Fig. 1, the interventional device is a biopsy needle and the planned trajectory is a planned needle trajectory. The data processing unit 16 is configured to adjust the planned needle trajectory according to the current motion of the subject. The data processing unit 16 is also configured to adjust the planned needle trajectory for at least one of the group of insertion point, trajectory angle and spatial depth.

For example, the insertion point relates to the location on the surface of the subject, where the device is inserted into the subject's body.

For example, the trajectory angle relates to the angle of the device, or the path of the device, in relation to the subject's body, or in relation to another geometric reference.

For example, the spatial depth relates to an insertion depth of the biopsy needle.

According to a further option, the motion of the at least one first reference marker is assigned to the first motion sub-model of the diaphragm and the motion of the at least one second reference marker is assigned to the second motion sub-model of the thorax.

The detected motion of the reference markers is transferred to the 3D motion model to generate model motion in order to simulate the current motion situation.

At least on reference marker is provided for one of the two main motion aspects, i.e. the ribcage and the diaphragm.

According to a further option, the 3D motion model is based on an elastic lung model that is adapted to the pre-operationally acquired image data of the subject.

In an example, the pre-operationally acquired image data comprises a single static 3D image. For example, the 3D image is taken at a defined breathing state such as maximum inhale. The range of motion can then be derived from anatomical constrains and/or from the measured traces.

In another example, the pre-operationally acquired image data comprises a sequence of images covering at least a part of the subject's lung motion by motion of the thorax and motion of the diaphragm.

The detected motion of the thorax, and the detected motion of the diaphragm are applied to an elastic lung model as constraints or boundary conditions for achieving an adapted subject-specific 3D motion model. As a basic constraint, the form of the subject's lung as detected, e.g. in CT or another imaging modality, is overlaid to the elastic model and the elastic model is adapted accordingly to match with the actual lung form.

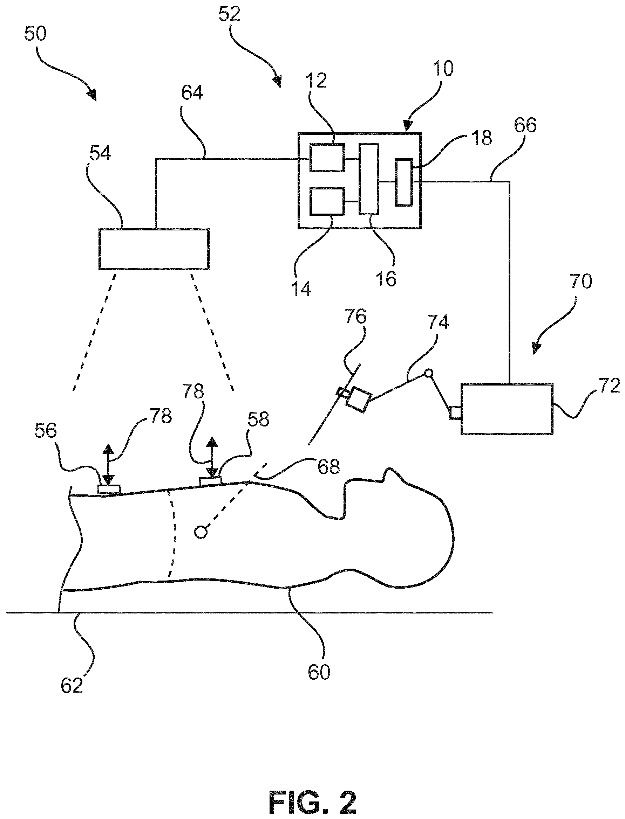

In an example, the lung model is provided as a subject patient specific lung model based on a subject's lung topography as derived from the imaging. For example, as generic aspects, elastic parameters used which are generally not accessible for individual patients can be provided. Fig. 2 shows an example of a guidance system 50 for lung intervention procedures. The system 50 comprises a device 52 for guidance in lung intervention procedures according to one of the preceding examples. Further, the system 50 comprises a marker tracking device 54 for tracking at least one first reference marker relating to the subject's abdomen area and at least one second reference marker relating to the subject's thorax area. The marker tracking device 54 is configured to track the reference markers and to provide the current tracking data.

As an option, it is further provided at least one first reference marker 56 relating to the subject's abdomen area, and at least one second reference marker 58 relating to the subject's thorax area.

In an option, as shown, reference markers are provided as external markers configured to be provided temporarily on the subject. In an example, the at least one first reference marker is configured to be provided on the subject relating to the subject's abdomen area, and the at least one second reference marker is configured to be provided on the subject relating to the subject's thorax area. The reference markers 56, 58 are shown attached to a subject 60 arranged on a subject support 62, e.g. a patient table.

In another option, not further shown, the reference markers are landmarks of a subject. The at least one first reference marker is a determined first landmark of the subject relating to the subject's abdomen area, and the at least one second reference marker is a determined second landmark of the subject relating to the subject's thorax area

In another example, not further shown, the reference markers are provided as reference landmarks in form of existing anatomical structures.

A data connection 64 is indicated for connecting, wire-based or wireless, the marker tracking device 54 with the input unit 12. A further data connection 66 is indicated for providing an adjusted trajectory 68 to further use, such as in form of path data.

In an example, also shown in Fig. 2 as an option, a handling device 70 for operating the interventional device is provided configured to move the interventional device according to path data, and the live-adjusted trajectory path is provided to the handling device as updated path data. The handling device may comprise a central base 72, to which a movable robotic arm 74 is connected. A gripping or holding portion 76 is mounted at a distal end of the robotic arm 74 for holding a biopsy needle for example.

Arrows 78 indicate motion of the reference markers due to e.g. breathing motion of the patient.

In an example, the reference markers are determined landmarks of the subject.

In a further option of the system 50, it is further provided a camera system with at least one camera 78 that tracks the reference markers.

For example, for the marker placement and biopsy procedure, the subject is continuously monitored by 3D cameras (e.g. within a surgical navigation interventional system from Philips) and a needle trajectory is provided for the physician, e.g. by projecting it onto the subject's chest or by depicting it on a nearby display.

For example, the reference markers are tracked by cameras arranged on lighting equipment in the operation or examination room. In an example, the cameras are provided above a region of interest of the subject. For example, the cameras are arranged above an area of a subject support like a patient table, which area is provided for supporting the upper body part of the subject, the upper body part comprising the subject's lung.

In another example, the reference markers are tracked by fluoroscopy imaging, e.g. with low dose X-ray radiation.

In a further example, a Light2C system is extended with a compact topographical mapping system. Provided that a 3D scanner is rigidly fixed to the Light2C system (or at least that the exact position of the 3D scanner with respect to the C-arm is known at all times), the imaging data (cone beam CT or fluoroscopy) can be directly registered to the topographical data of the subject body.

For example, a plurality of time of flight cameras is provided to track the spatial arrangement of the subject and thus the motion of the subject.

In a further option, not shown in detail, an X-ray imaging device is provided and the reference markers are tracked by the X-ray imaging device.

This example does not use cameras or additional markers. Instead, X-ray fluoroscopy is used e.g. in the form of a C-arm to continuously monitor internal markers (e.g. the ribs, the outline of the lungs, etc.). This has the additional advantage that the diaphragm position itself could also be tracked with the X-ray system.

Alternatively, or in addition to the option in which reference markers are external reference markers, in another option, the reference markers are pre-determined anatomical reference landmarks. In an example, artificial markers are not used. Instead, (static and moving) anatomical markers are used which are visible in the camera field of view. For improving the identification and detection in the image, cameras are provided with, for instance, a large optical bandwidth such as hyperspectral imaging systems which record images in both the visible and near-infrared wavelength range. Such camera systems can record additional anatomical features that are not directly visible to the eye.

In another example, a surface scanning method that is capable of providing a real-time, 3D topographic representation of the subject body surface (mainly thoracic and abdominal part) is provided, e.g. in combination with cameras, but also without cameras.

In an option, a projecting device is provided configured to project the live-adjusted trajectory path to the user.

For example, the projecting device projects the live-adjusted trajectory path onto the subject.

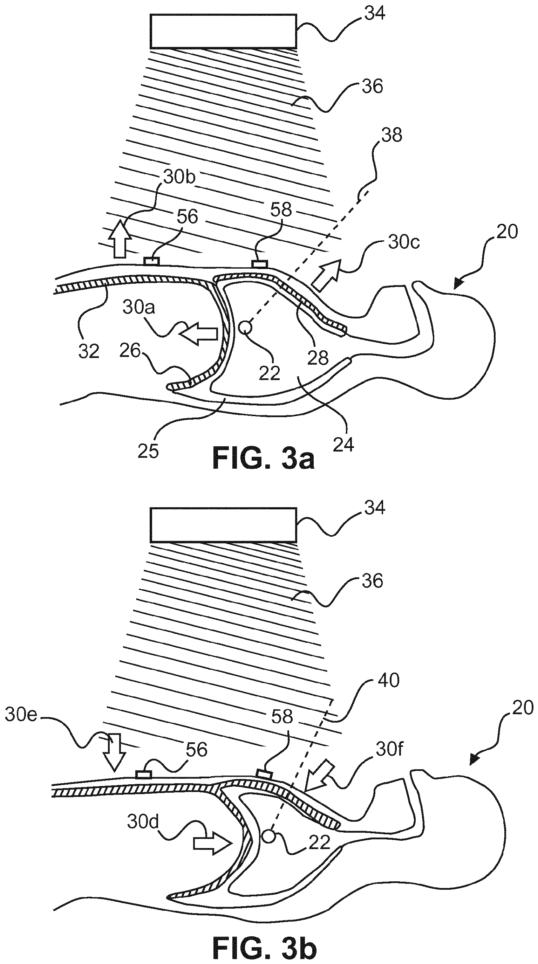

Fig. 3a and 3b show schematic illustrations relating to motion due to breathing, wherein Fig. 3a refers to an initial state and Fig. 3b refers to a moved state with indicated adjusted trajectory.

In Fig. 3a, a subject 20 is indicated with a point or location of interest 22 inside a lung 24 of the subject 20, at least partly surrounded by a pleural cavity 25. Further, a diaphragm 26 and a rib structure 28 of the subject 20 are schematically indicated. A motion of the lung 24 is caused by breathing, which in turn is actuated by the movement of the diaphragm 26 and the rib structure 28. A first arrow 30a indicates the motion of the diaphragm 26 when inhaling. A second arrow 30b indicates the motion of an abdomen area 32 when inhaling. A third arrow 30c indicates the motion of the rib structure 28 when inhaling. The resulting motion is tracked by the least one first reference marker 56 relating to the subject's diaphragm and the at least one second reference marker 58 relating to the subject's thorax area.

For tracking the reference markers 56, 58, an example of a Light2C system 34 is provided where cameras track (indicated with tracking field boundary lines 36) the reference markers 56, 58. As an initial state, a planned needle trajectory 38 is indicated.

In Fig. 3b, an exhaling state is indicated. A fourth arrow 30d indicates the motion of the diaphragm 26 when inhaling. A fifth arrow 30e indicates the motion of the abdomen area 32 when exhaling. A sixth arrow 30f indicates the motion of the rib structure 28 when inhaling. The resulting motion is tracked by the least one first reference marker 56 and the at least one second reference marker 58. Instead of the planned trajectory, a live-adjusted trajectory path 40 is provided.

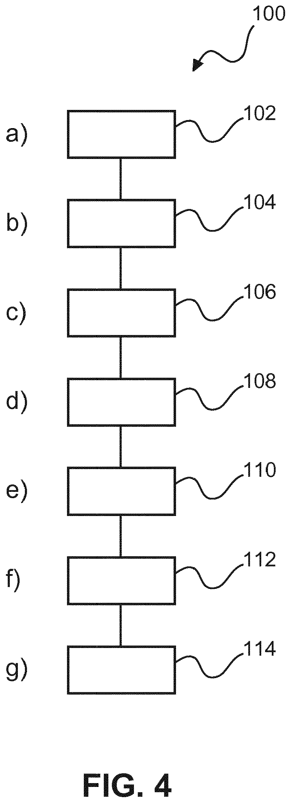

Fig. 4 shows an example of a method 100 for guidance in lung intervention procedures. The method 100 comprises the following steps:
- In a first step 102, also referred to as step a), a 3D motion model of a torso of a subject is provided comprising at least the lung and a part of the abdomen area. The 3D motion model is based on pre-operationally acquired image data of the subject.
- In a second step 104, also referred to as step b), at least one first reference marker on the subject is identified relating to the subject's abdomen area, and at least one second reference marker on the subject is identified relating to the subject's thorax area.
- In a third step 106, also referred to as step c), the reference markers are registered with the 3D motion model.
- In a fourth step 108, also referred to as step d), the at least one first reference marker and the at least one second reference marker are tracked to determine a current motion of the reference markers.
- In a fifth step 110, also referred to as step e), the current motion of the reference markers is transferred to the 3D motion model and the 3D motion model is adapted based on the transferred current motion. The positions of predetermined lung structures are adjusted within the 3D motion model.
- In a sixth step 112, also referred to as step f), a planned trajectory of an interventional device is adjusted according to the adjusted positions to provide a live-adjusted trajectory path.
- In a seventh step 114, also referred to as step g), the adjusted trajectory is provided for the lung intervention procedure.
In an example, the 3D motion model comprises a first motion sub-model of the diaphragm and a second motion sub-model of the thorax.

In an example, the first step 102 is provided as two sub-steps. In a first sub-step, also referred to as step a1), 3D image data is acquired. In a second sub-step, also referred to as step a2), the system uses the 3D image data to generate the 3D motion model, respectively the motion models, i.e. the first motion sub-model of the diaphragm and the second motion sub-model of the thorax. The model generation may take place with or without additional input from an operator.

In an example, step c) takes before step d). In an alternative example, step c) takes place after step d).

In an example, in step c), an algorithm determines the spatial position of the markers from the camera images and uses it to register the pre-procedural 3D image(s) and the camera image. Further, in step f), the lung volume is adjusted as necessitated by the breathing state and the subject position. This step makes use of a lung-deformation-during-breathing model.

In step e), the position of important lung structures (lesions, major blood vessels) is adjusted based on the lung deformation.

In an example, for step e), since the markers are registered with the 3D motion model, it is possible to track their current motion and to transfer the motion to the model, as the reference markers and the 3D motion model are registered and thus linked together. The 3D motion model is then adapted accordingly. This also changes the positions of predetermined lung structures like lesions or the like within the 3D motion model, which positions are hence adjusted.

In step f), the trajectory of the marker or biopsy needle is updated in real time based on the new location information and the needle location so that the device can reach the target while avoiding sensitive structures.

To track the location of a lesion via the tracking of the markers and transferring the motion to the motion model to then determine the current position of the lesion, provides biopsy guidance that is also suitable for targeting a lesion in the lower part of the lung.

The use of optical cameras eliminates the need for e.g. real-time fluoroscopy. Hence, cost or radiation dose benefits are provided by the optical cameras.

The tracking of the markers and adapting a 3D model, results in optimized trajectories compared to trajectories based on static subject anatomy.

In an example, indicated in Fig. 3 as an option, the live-adjusted trajectory path is i) projected to the user, and or ii) provided to a handling device for operating the interventional device.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for guidance in a lung intervention procedure, the device comprising:
- an input unit (12);
- a data storage unit (14);
- a data processing unit (16); and
- an output unit (18);
wherein the input unit is configured to provide current tracking data of at least one first reference marker relating to a subject's abdomen area and of at least one second reference marker relating to the subject's thorax area;
wherein the data storage unit is configured to provide a 3D motion model of a torso of the subject comprising at least the lung and a part of the abdomen area; and wherein the 3D motion model is based on pre-operationally acquired image data of the subject;
wherein the data processing unit is configured to register the reference markers with the 3D motion model; to determine a current motion of the reference markers; to transfer the current motion of the reference markers to the 3D motion model; to adapt the 3D motion model based on the transferred current motion; to adjust positions of predetermined lung structures within the 3D motion model; and to adjust a planned trajectory of an interventional device according to the adjusted positions to provide a live-adjusted trajectory path; and
wherein the output unit is configured to provide the adjusted trajectory for the lung intervention procedure.

2. Device according to claim 1, wherein the 3D motion model comprises a first 3D motion sub-model of the diaphragm and a second motion sub-model of the thorax; and
wherein the data processing unit is configured to: transfer the current motion of the at least one first reference marker relating to the subject's abdomen area to the first 3D motion sub-model of the diaphragm and to transfer the current motion of the at least one second reference marker relating to the subject's thorax area to the second 3D motion sub-model of the thorax; to adapt the first and second 3D motion sub-models based on the transferred current motion; to adjust the positions of predetermined lung structures within the 3D motion sub-models; and to adjust the planned trajectory of an interventional device according to the adjusted positions to provide the live-adjusted trajectory path.

3. Device according to claim 1 or 2, wherein it is further provided:
- a model-generating unit;
wherein the model-generating unit is configured to generate motion models based on 3D image data by segmenting moving structures and assigning mechanical properties and boundary conditions to the moving structures.

4. Device according to claim 1, 2 or 3, wherein the interventional device is a biopsy needle and the planned trajectory is a planned needle trajectory; and
wherein the data processing unit is configured to adjust the planned needle trajectory according to the current motion of the subject; and to adjust the planned needle trajectory for at least one of the group of insertion point, trajectory angle and spatial depth.

5. Device according to one of the preceding claims, wherein the 3D motion model is based on an elastic lung model that is adapted to the pre-operationally acquired image data of the subject.

6. A guidance system (50) for lung intervention procedures, the system comprising:
- a device (52) for guidance in lung intervention procedures according to one of the preceding claims; and
- a marker tracking device (54) for tracking at least one first reference marker relating to the subject's abdomen area and at least one second reference marker relating to the subject's thorax area;
wherein the marker tracking device is configured to track the reference markers and to provide the current tracking data.

7. System according to claim 6, wherein it is further provided:
- at least one first reference marker (56) relating to the subject's abdomen area; and
- at least one second reference marker (58) relating to the subject's thorax area;
wherein the reference markers are external markers configured to be provided temporarily on the subject; wherein the at least one first reference marker is configured to be provided on the subject relating to the subject's abdomen area, and the at least one second reference marker is configured to be provided on the subject relating to the subject's thorax area.

8. System according to claim 7, wherein it is further provided:
- a camera system with at least one camera (68);
wherein the reference markers are tracked by the at least one camera.

9. System according to one of the claims 6 to 8, wherein it is further provided:
- an X-ray imaging device;
wherein the reference markers are tracked by the X-ray imaging device.

10. System according to one of the claims 6 to 9, wherein a projecting device is provided configured to project the live-adjusted trajectory path to the user.

11. System according to one of the claims 6 to 10, wherein a handling device (78) for operating the interventional device is provided configured to move the interventional device according to path data, and the live-adjusted trajectory path is provided to the handling device as updated path data.

12. A method (100) for guidance in lung intervention procedures, the method comprising the following steps:
a) providing (102) a 3D motion model of a torso of a subject comprising at least the lung and a part of the abdomen area,; wherein the 3D motion model is based on pre-operationally acquired image data of the subject;
b) identifying (104) at least one first reference marker on the subject relating to the subject's abdomen area; and identifying at least one second reference marker on the subject relating to the subject's thorax area;
c) registering (106) the reference markers with the 3D motion model;
d) tracking (108) the at least one first reference marker and the at least one second reference marker to determine a current motion of the reference markers;
e) transferring (110) the current motion of the reference markers to the 3D motion model and adapting the 3D motion model based on the transferred current motion; and adjusting positions of predetermined lung structures within the 3D motion model;
f) adjusting (112) a planned trajectory of an interventional device according to the adjusted positions to provide a live-adjusted trajectory path; and
g) providing (114) the adjusted trajectory for the lung intervention procedure.

13. Method according to claim 12, wherein the live-adjusted trajectory path is:
i) projected to the user; and or
ii) provided to a handling device for operating the interventional device.

14. A computer program element for controlling an apparatus according to one of the claims 1 to 11, which, when being executed by a processing unit, is adapted to perform the method steps of one of the claims 12 to 13.

15. A computer readable medium having stored the program element of claim 14.
